(19) Europäisches Patentamt
European Patent Office
Office européen des brevets

(11) Veröffentlichungsnummer: **0 019 764**
**A1**

# (12) EUROPÄISCHE PATENTANMELDUNG

(21) Anmeldenummer: **80102509.9**

(22) Anmeldetag: **08.05.80**

(51) Int. Cl.³: **C 07 D 211/14**, C 07 D 265/30, A 61 K 31/445, A 61 K 31/535

(30) Priorität: **25.05.79 DE 2921221**

(43) Veröffentlichungstag der Anmeldung: **10.12.80 Patentblatt 80/25**

(84) Benannte Vertragsstaaten: **AT BE CH DE FR GB IT LI LU NL SE**

(71) Anmelder: **BASF Aktiengesellschaft, Carl-Bosch-Strasse 38, D-6700 Ludwigshafen (DE)**

(72) Erfinder: **Himmele, Walter, Dr.Dipl.-Chem., Eichenweg 14, D-6909 Walldorf (DE)**
Erfinder: **Heberle, Wolfgang, Dr.Dipl.-Biol., An der Düne 4, D-2082 Moorrege (DE)**
Erfinder: **Kohlmann, Wilhelm, Dr., An der Düne 6, D-2082 Moorrege (DE)**
Erfinder: **Wesenberg, Walter, Dorfstrasse 16, D-2421 Bujendorf über Eutin (DE)**

(54) **Trans-3-(4'-tert.-Butyl-cyclohexyl-1')-2-methyl-1-(3'-methylpiperidino, 3',5'-dimethylpiperidino und 2', 6'-dimethylmorpholino)-propan, Verfahren zu ihrer Reinherstellung, diese enthaltende antimykotische Mittel und ihre Verwendung.**

(57) Die Erfindung betrifft reines trans-3-(4'-tert.-Butyl-cyclohexyl-1')-2-methyl-1-(3'-methyl-1'-piperidino)-propan, trans-3-(4'-tert.-Butyl-cyclohexyl-1')-2-methyl-1-(3',5'-dimethyl-1'-piperidino)-propan und trans-3-(4'-tert.-Butyl-cyclohexyl-1')-2-methyl-1-(2',6'-cis-dimethylmorpholino)-propan, ihre Reinherstellung aus cis/trans-Isomerengemischen, diese enthaltende antimykotische Mittel zur topischen und systematischen Anwendung und ihre Verwendung bei der Bekämpfung von Dermatomykosen.

EP 0 019 764 A1

trans-3-(4'-tert.-Butyl-cyclohexyl-1')-2-methyl-1-(3'-methylpiperidino, 3',5'-dimethylpiperidino und 2',6'-dimethylmorpholino)-propan, Verfahren zu ihrer Reinherstellung, diese enthaltende antimykotische Mittel und ihre Verwendung

Aus der DE-OS 27 52 135 ist bekannt, daß 3-(4'-tert.-Butyl-cyclohexyl-1')-2-methyl-1-(3'-methyl-1'-piperidino)-propan, 3-(4'-tert.-Butyl-cyclohexyl-1')-2-methyl-1-(3',5'-dimethyl-1'-piperidino)-propan und 3-(4'-tert.-Butyl-cyclohexyl-1')-2-methyl-1-(2',6'-cis-dimethyl-morpholino)-propan und ihre Salze zur Bekämpfung von pflanzenpathogenen Pilzen und auch von humanpathogenen Pilzen und Hefen, wie beispielsweise Candida albicans, Trichophyton mentagrophytes und Histoplasma capsulatum, verwendet werden können.

Die genannten Verbindungen liegen im Hinblick auf die beiden Substituenten am Cyclohexanring in 1,4-Stellung als cis-trans-Isomerengemische vor.

Die vorliegende Erfindung betrifft die reinen trans-Verbindungen

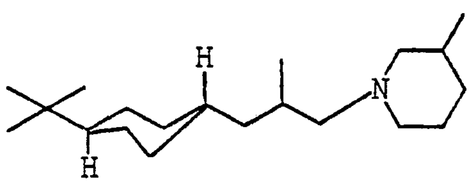

(1)

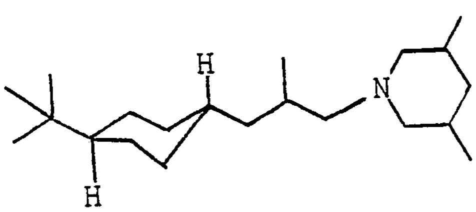

(2)

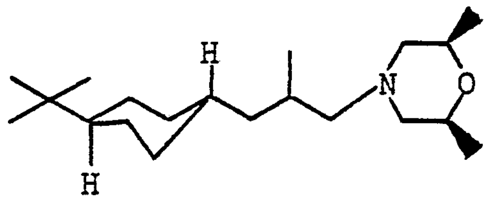

(3)

trans-3-(4'-tert.-Butyl-cyclohexyl-1')-2-methyl-1-(3'-methyl-1'-piperidino)-propan (1),
trans-3-(4'-tert.-Butyl-cyclohexyl-1')-2-methyl-1-(3',5'-dimethyl-1'-piperidino)-propan (2)
und trans-3-(4'-tert.-Butyl-cyclohexyl-1')-2-methyl-1-(2',6'-cis-dimethylmorpholino)-propan (3),
die Reinherstellung aus cis/trans-Isomerengemischen und ihre Verwendung in antimykotischen Mitteln.

Aufgrund der überraschenden Feststellung, daß die reinen trans-Verbindungen gegenüber humanpathogenen Pilzen und Hefen aus dem Verwandtschaftskreis der Dermatophyten um das 10-fache und noch darüber hinaus stärker wirksam sind als die cis-Verbindungen, ist es eine Aufgabe der Erfindung, Verfahren zur Herstellung der reinen trans-Verbindungen (1) bis (3) zu entwickeln.

Der Fachmann weiß, daß bei der katalytischen Hydrierung von para-substituierten Aromaten Isomerengemische verschiedenster Zusammensetzungen entstehen. Beispielsweise entstehen häufig überwiegend cis-1,4-disubstituierte Cyclohexanverbindungen an Platinkatalysatoren, auch mit Nickel-, Kobalt- oder Palladium-Katalysatoren durchgeführte Hydrierungen führen überwiegend zu den cis-disubstituierten Cyclohexanverbindungen (vgl. Houben-Weyl, Bd. 11/1,

S. 680 ff, Georg Thieme-Verlag, Stuttgart 1957). Mit Ruthenium-Katalysatoren werden ähnliche Ergebnisse erzielt, es werden jedoch auch Anteile der entsprechenden trans--Verbindungen erhalten.

Bei der Reduktion von para-substituierten Aromaten mit Natrium in Alkohol werden im allgemeinen deutlich mehr trans-Anteile erhalten, jedoch ist die Selektivität dieser Reaktion nicht befriedigend und der technische Aufwand wesentlich höher.

Weiterhin ist bekannt, beispielsweise aus Houben-Weyl, Bd. 5/1a, S. 553 ff, Georg Thieme-Verlag, Stuttgart 1970), daß die Auftrennung eines Gemisches aus cis/trans-1,4-disubstituierten Cyclohexanverbindungen außerordentlich stark wirksame Kolonnen erfordert. Meist werden Trennaggregate von über 100 theoretischen Böden eingesetzt und ein sehr hohes Rücklaufverhältnis von 1 : 200 gewählt. Die großen Schwierigkeiten bei der Auftrennung von cis--trans-Gemischen bei disubstituierten Cyclohexanderivaten gehen beispielsweise daraus hervor, daß für die Auftrennung von 1,4-Diisopropyl-cyclohexan in das cis- und trans-Isomere eine Kolonne von 100 theoretischen Trennstufen benötigt wird, wobei mit einem Rücklaufverhältnis von 1 : 200 gearbeitet wird. Das reine cis-Isomere siedet bei 14 Torr bei 96,9°C und das trans-Isomere bei 96,1°C. Wird die Fraktionierung bei einem Druck von 7,5 Torr vorgenommen, so siedet das cis-Isomere niedriger, und zwar bei 78,9°C, und das trans-Isomere bei 84,4°C. Dieses Umschlagen der Siedepunkte beschreibt sehr deutlich die Schwierigkeiten einer solchen Trennung.

Die Trennung des 1,4-Di-tert.-butyl-cyclohexans in die entsprechenden reinen cis- und trans-Isomeren erfordert etwa die gleichen Trennbedingungen. Es werden, wie die

Versuchsbeschreibungen zeigen, mehrere Tage benötigt, um kleine Mengen der reinen Verbindungen zu isolieren (Houben-Weyl, Bd. 5/1a, S. 558). Ebenso wird gezeigt, daß auch die Trennung des Gemisches der isomeren Formen, das durch Hydrierung von 1-Methyl-4-tert.-butyl-benzol erhalten wird, einen sehr hohen Aufwand erfordert.

Diese Beispiele zeigen, daß eine Trennung von 1,4-disubstituierten Cyclohexanderivaten außerordentlich aufwendig und schwierig ist und bei entsprechender Vergrößerung eines Substituenten, wie sie bei den oben genannten Verbindungen vorliegen, eine Auftrennung in die reine cis-Isomere und trans-Verbindung durch fraktionierte Destillation technisch nicht mehr erwartet werden kann.

Es wurde nun gefunden, daß man die reinen trans-3-(4'-tert.-Butyl-cyclohexyl-1')-2-methyl-1-(3'-methyl-piperidino, 3',5'-dimethylpiperidino und 2',6'-cis-dimethylmorpholino)-propane erhält, wenn man die bei der Hydrierung der entsprechenden 1,4-disubstituierten Phenylverbindung in an sich üblicher Weise zur Cyclohexanverbindung erhaltenen cis-trans-Gemische nach dem Abdestillieren des für die Hydrierung verwendeten Lösungsmittels durch eine fraktionierte Destillation bei einem Druck am Kolonnenkopf von 0,05 bis 50 torr an einer 10- bis 100-bödigen Kolonne unter einem Rücklaufsverhältnis von 1 : 1 bis 1 : 100 und bei den bei der Destillation sich einstellenden Temperaturen auf einen trans-Anteil von über 85 % anreichert und das reine trans-Isomere nach Umwandlung in ein Säureadditionssalz an Halogenwasserstoffsäure und Kristallisation in einem organischen Lösungsmittel auskristallisieren läßt.

Die bei der fraktionierten Destillation am Kolonnenkopf gemessenen Temperaturen, die sich aufgrund der Rücklaufverhältnisse und Zahl der Böden einstellen, liegen in der Regel bei 160 bis 240°C.

Überraschender Weise werden die oben genannten trans-Cyclohexanverbindungen (1), (2) und (3) gegenüber den beschriebenen schwierigen Trennungen von 1,4-Dialkyl-cyclohexan mit wesentlich geringerem Aufwand an Destillationstechnik getrennt, obwohl das Molekül eine tertiäre Aminogruppe aufweist, in niedrigsiedende mit der cis-Verbindung angereicherte und in höhersiedende mit der trans-Verbindung angereicherte Fraktionen aufgetrennt.

In der Regel werden Isomerengemische fraktioniert destilliert, die erhalten worden sind durch Hydrierung in an sich üblicher Weise an einem Ruthenium-, Palladium-, Platin-, Nickel- oder Kobalt-Katalysator.

Als Kolonnen sind Glassiebbodenkolonnen oder mit Silber- oder Edelstahldrahtnetzwendeln gefüllte Kolonnen zweckmäßig.

Die bevorzugten Bedingungen für die fraktionierte Destillation von (1) sind eine 20- bis 40-bödige Kolonne bei 2 bis 20 torr und einem Rücklaufverhältnis von 1 : 3 bis 1 : 10, wobei sich Temperaturen von 183 bis 186°C einstellen;

die für (2) sind eine 20- bis 40-bödige Kolonne bei 2 bis 20 torr und einem Rücklaufverhältnis von 1 : 3 bis 1 : 10, wobei sich Temperaturen von 193 bis 200°C einstellen und

die für (3) sind eine 20- bis 40-bödige Kolonne bei 2 bis 20 torr und ein Rücklaufverhältnis von 1 : 3 bis 1 : 10, wobei sich Temperaturen von 203 bis 208°C einstellen.

Dabei kann nach Beendigung der Destillation ein Destillationsrückstand von 5 bis 10 % verbleiben, der, einer Kurzweg-Destillation unter den oben angegebenen Bedingungen unterworfen, bereits Reinheitsgrade von 92 bis 98 % an trans-Verbindung liefert.

Die auf über 85 % an trans-Verbindung angereicherten Fraktionen werden in einem geeigneten Lösungsmittel und nach Umwandlung in ein Säureadditionssalz einer Halogenwasserstoffsäure durch Kristallisation in die reine trans-Verbindung überführt.

Geeignete Lösungsmittel für die Kristallisation sind insbesondere einwertige niedere Alkohole mit 1 bis 4 C-Atomen, wie Methanol und Äthanol, Ester niederer aliphatischer Carbonsäuren mit 1 bis 4 C-Atomen im Esteralkohol und in der Säure, insbesondere Essigester, wie Essigsäureäthylester, niedere Ketone, wie Aceton, Methyläthylketon und Methylisopropylketon, oder Dialkyläther mit 1 bis 4 C-Atomen im Alkyl oder gesättigte cyclische Äther, wie Diäthyläther, Diisopropyläther, Tetrahydrofuran und Dioxan. Gegebenenfalls erfolgt die Kristallisation in Gegenwart überschüssiger Mengen von Halogenwasserstoffsäure. Das bevorzugte Säureadditionssalz ist das Hydrochlorid.

Bevorzugte Kristallisationsbedingungen sind etwa 20 gewichtsprozentige methanolische oder äthanolische Lösungen, die bei etwa 80°C hergestellt werden und die man auf etwa 25°C abkühlen läßt, gegebenenfalls unter Animpfen.

Die endgültige Abtrennung der reinen trans-Verbindung durch Kristallisation aus einem Isomerengemisch mit wenigstens 85 % trans-Anteil, hat den besonderen Vorteil, daß die vorausgehende fraktionierte Destillation weniger aufwendig betrieben zu werden braucht. Das führt zu einer geringeren thermischen Belastung der zu trennenden Isomeren, so daß Zersetzungsprodukte, die unter Umständen auftreten können, vermieden werden. Durch die Kombination von fraktionierter Destillation und Kristallisation werden in technisch überraschender einfacher Weise über 99 % reine trans-Verbindungen und auch die reinen cis-Verbindungen, wie die Ausführungsbeispiele zeigen, erhalten.

Die Mutterlaugen können nach Freisetzung der Base erneut einer fraktionierten Destillation unterworfen werden.

Es wird noch darauf hingewiesen, daß man die cis-Verbindung in ihren Gemischen oder nach erfindungsgemäßer Abtrennung der trans-Verbindung durch eine Palladium- oder Nickel--katalysierte Isomerisierung in die trans-Verbindung überführen kann.

Die erfindungsgemäße Trennung und Herstellung der reinen trans-Isomeren schafft als weiteren hervorzuhebenden Vorteil die Voraussetzung, daß die aufgrund der vorhandenen Chiralitätszentren existierenden Konfigurationsisomere in die optischen Antipoden getrennt werden können.

Überraschenderweise sind die reinen trans-Verbindungen fast ausschließlich Träger der guten antimykotischen Wirkung. Verglichen mit der jeweiligen cis-Verbindung und bezogen auf die Hemmeffekte gegenüber Mikroorganismen, sind die trans-Verbindungen (1), (2) und (3) 10-fach und noch darüber hinaus stärker wirksam. Das bedeutet beispielsweise bei etwa gleichen Toxizitätswerten eine wesentliche Verbesserung der therapeutischen Breite.

Gegenstand der vorliegenden Erfindung ist daher auch ein antimykotisches Mittel zur topischen und systemischen Anwendung, gekennzeichnet durch einen Gehalt an einer reinen trans-Verbindung gemäß Patentansprüchen 1 bis 3 oder deren physiologisch verträglichen Säureadditionssalz als Wirkstoff neben üblichen Träger- und Verdünnungsmitteln sowie die Verwendung der reinen trans-Verbindung bei der Bekämpfung von Dermatomykosen.

Zur Salzbildung mit einer an sich üblichen physiologisch verträglichen Säure kommen organische oder anorganische Säuren, wie Salpetersäure, Salzsäure, Schwefelsäure, Phosphorsäure, Essigsäure, Propionsäure, Milchsäure, Bernsteinsäure, Weinsäure, Zitronensäure, Benzoesäure, Salicylsäure oder Nicotinsäure in Betracht. Bevorzugt werden jedoch die genannten anorganischen Säuren, insbesondere Salzsäure.

Die erfindungsgemäß zu verwendenden reinen trans-Verbindungen und ihre Salze weisen starke antimykotische Wirkungen auf. Sie besitzen ein breites antimykotisches Wirkungsspektrum, insbesondere gegen Dermatophyten, z.B. Epidermophyton-Arten, wie Epidermophyton floccosum, Trichophyton-Arten, wie Trichophyton mentagrophytes, Microsphoron-Arten, wie Microsphoron ferrugineum. Die Aufzählung dieser Mikroorganismen soll keine Beschränkung der zu bekämpfenden Mikroorganismen darstellen, sondern nur zur Erläuterung dienen.

Die Wirkung gegenüber Mikroorganismen kann nach Methoden, wie sie beispielsweise in P. Klein, Bakteriologische Grundlagen der chemotherapeutischen Laboratoriumspraxis, Springer-Verlag Berlin, 1957, beschrieben sind, aufgezeigt werden.

Beispielsweise wurde geprüft, welche minimalen Hemmkonzentrationen (MHK-Werte) im Agardilutionstest gegen pathogene Pilze erreicht werden können.

In der folgenden Tabelle 1 sind die Ergebnisse zusammengefaßt:

Tabelle 1 - Vergleichende MHK-Werte (µg/ml) bei Dermatophyten von Cyclohexylpropylamin-Derivaten, Clotrimazol, Miconazol und Tolnaftat

| Spezies | Stamm | Verbindung (2) cis | (2) trans ++) | (3) cis | (3) trans | (1) cis | (1) trans |
|---|---|---|---|---|---|---|---|
| Microsporum ferrugineum | No 5/M+) | 0,0625 | <0,0078 | 0,0312 | <0,0078 | 0,0312 | 0,0078 |
| Microsporum gypseum | No 156/M | 0,125 | 0,0312 | 0,125 | 0,0156 | 0,25 | 0,0156 |
| Microsporum canis | No 155/M | 0,125 | 0,0312 | 0,5 | 0,0312 | 0,125 | 0,0156 |
| Microsporum canis | No 167/M | 0,125 | 0,0156 | 0,0625 | 0,0312 | 0,25 | 0,0312 |
| Microsporum canis | No 168/M | 0,0312 | <0,0078 | 0,0625 | 0,0156 | 0,0625 | 0,0156 |
| Microsporum canis | No 169/M | 0,125 | 0,0312 | 0,125 | 0,0312 | 0,5 | 0,0625 |
| Trichophyton schoenleinii | No 9/M | 0,0625 | 0,0156 | 0,125 | 0,0156 | 0,125 | 0,0625 |
| Trichophyton violaceum | No 10/M | 0,0625 | <0,0078 | 0,125 | 0,0156 | 0,0625 | 0,0156 |
| Trichophyton rubrum | No 158/M | 0,125 | 0,0312 | >1 | 0,0625 | 0,25 | 0,0312 |
| Trichophyton mentagrophytes | No 19/M | 0,25 | 0,0625 | 1 | 0,0312 | 0,5 | 0,0312 |
| Trichophyton mentagrophytes | No 21/M | 0,125 | 0,0312 | 1 | 0,0625 | 0,25 | 0,0312 |
| Trichophyton mentagrophytes | No 159/M | 0,0156 | <0,0078 | 0,0625 | <0,0078 | 0,0312 | 0,0078 |
| Trichophyton mentagrophytes | No 160/M | 0,125 | 0,0312 | 0,125 | 0,0625 | 0,25 | 0,0312 |
| Trichophyton mentagrophytes | No 170/M | 0,0625 | <0,0078 | 0,0625 | 0,0156 | 0,125 | 0,0156 |
| Trichophyton mentagrophytes | No 171/M | 0,0625 | <0,0078 | 0,0625 | 0,0156 | 0,125 | 0,0156 |
| Trichophyton mentagrophytes | No 172/M | 0,125 | 0,0312 | 0,25 | 0,0625 | 0,5 | 0,0625 |
| Trichophyton mentagrophytes | No 173/M | 0,25 | 0,0312 | 0,25 | 0,0625 | 0,25 | 0,0312 |
| Trichophyton mentagrophytes | No 174/M | 0,125 | 0,0156 | 0,125 | 0,0156 | 0,125 | 0,0312 |
| Epidermophyton floccosum | No 157/M | 0,125 | 0,0156 | 0,25 | 0,0312 | 0,25 | 0,0312 |

+) No 5/M = Stamm 5 der Mykothek, Nordmark-Werke GmbH

++) (2) trans als Hydrochlorid, alle übrigen Verbindungen als freie Base

Fortsetzung Tabelle 1

| Spezies | Stamm | Clotrimazol | Miconazol | Tolnaftat |
|---|---|---|---|---|
| Microsporum ferrugineum | No 5/M+) | 0,16 | > 1,28 | 0,16 |
| Microsporum gypseum | No 156/M | 0,64 | > 1,28 | 0,16 |
| Microsporum canis | No 155/M | 0,32 | > 1,28 | 0,32 |
| Microsporum canis | No 167/M | 0,64 | > 1,28 | 0,16 |
| Microsporum canis | No 168/M | 0,16 | > 1,28 | 0,04 |
| Microsporum canis | No 169/M | 0,64 | > 1,28 | 0,32 |
| Trichophyton schoenleinii | No 9/M | 0,64 | > 1,28 | 0,04 |
| Trichophyton violaceum | No 10/M | 0,16 | 0,64 | < 0,01 |
| Trichophyton rubrum | No 158/M | 1,28 | > 1,28 | 0,08 |
| Trichophyton mentagrophytes | No 19/M | 1,28 | > 1,28 | 0,04 |
| Trichophyton mentagrophytes | No 21/M | 1,28 | > 1,28 | 0,32 |
| Trichophyton mentagrophytes | No 159/M | 0,32 | 0,32 | 0,08 |
| Trichophyton mentagrophytes | No 160/M | 0,64 | > 1,28 | 0,32 |
| Trichophyton mentagrophytes | No 170/M | 0,32 | 1,28 | 0,08 |
| Trichophyton mentagrophytes | No 171/M | 0,32 | 0,64 | 0,16 |
| Trichophyton mentagrophytes | No 172/M | 0,64 | 0,32 | 0,64 |
| Trichophyton mentagrophytes | No 173/M | 0,64 | 0,32 | 0,32 |
| Trichophyton mentagrophytes | No 174/M | 1,28 | > 1,28 | 0,08 |
| Epidermophyton floccosum | No 157/M | 0,32 | > 1,28 | 0,32 |

+) No 5/M = Stamm 5 der Mykothek, Nordmark-Werke GmbH

Aus der Tabelle 1 geht hervor, daß die trans-Verbindungen gegenüber den cis-Verbindungen wesentlich stärker wirken und somit praktisch Träger der Wirksamkeit sind. Weiterhin wird gezeigt, daß die trans-Verbindungen gegenüber den bekannten antimykotischen Mitteln Clotrimazol, Miconazol und insbesondere gegenüber Tolnaftat, eines der derzeit am meisten angewendeten Mitteln bei Dermatomykosen, deutlich niedere MHK-Werte aufweisen.

Der $LD_{50}$-Wert, ermittelt an der Maus per os, beträgt für (1)-trans 305 mg/kg, für (2)-trans als Hydrochlorid 335 mg/kg und für (3)-trans 2250 mg/kg. Aus diesen $LD_{50}$-Werten ergibt sich für die topische und systemische Anwendung nach oraler Gabe eine genügend therapeutische Breite.

Dementsprechend kommen als Indikationsgebiete an Mensch und Tier Dermatomykosen, insbesondere verursacht durch Species der Gattungen Epidermophyton, Microsporum und Trichophyton in Betracht.

Zur vorliegenden Erfindung gehört auch die Herstellung der Mittel oder Zubereitungen zur oralen und topischen Anwendung, die mit üblichen Trägerstoffen oder Verdünnungsmitteln und den üblicherweise verwendeten pharmazeutisch-technischen Hilfsstoffen mit einer zur innerlichen oder äußerlichen Anwendung geeigneten Dosierung in üblicher Weise, insbesondere durch Vermischen, erfolgt.

In der Regel hat es sich sowohl in der Human- als auch in der Veterinärmedizin als vorteilhaft erwiesen, den oder die Wirkstoffe in Mengen von etwa 0,07 bis 0,1 g/$cm^2$ Körperoberfläche je 24 Stunden, gegebenenfalls in Form mehrerer Einzelgaben, äußerlich zur Anwendung zu bringen. Es kann jedoch auch von den genannten Dosierungen abgewichen

werden, und zwar in Abhängigkeit von der Art und der Schwere der Erkrankung. Die Festlegung der jeweils erforderlichen optimalen Dosierung und deren Häufigkeit kann durch den behandelnden Arzt aufgrund seines Wissens variiert werden.

In der Regel werden die Einzelgaben 1 bis 2 mal täglich aufgebracht. Bei äußerlicher, lokaler Anwendung enthalten die Zubereitungen 0,5 bis 5, bevorzugt 1 bis 2 Gew.% Wirkstoff. Bei oraler Gabe kommen als Einzeldosierungen Mengen von 50 bis 1 000 mg, bevorzugt, 500 bis 800 mg, in Betracht, die beispielsweise täglich 1 bis 2 mal verabreicht werden.

Zubereitungen für die orale Applikation sind beispielsweise Tabletten, Filmtabletten, Dragees, Kapseln, Pillen, Pulver oder Suspensionen.

Entsprechende Tabletten können beispielsweise durch Mischen des Wirkstoffs mit bekannten Hilfsstoffen, beispielsweise inerten Verdünnungsmitteln, wie Dextrose, Zucker, Sorbit, Mannit, Polyvinylpyrrolidon, Calciumcarbonat, Calciumphosphat oder Milchzucker, Sprengmitteln, wie Mais, Stärke oder Alginsäure, Bindemitteln wie Stärke oder Gelantine, Gleitmitteln, wie Magnesiumstearat oder Talk, und/oder Mitteln zur Erzielung des Depoteffektes, wie Carboxypolymethylen, Carboxymethylcellulose, Celluloseacetatphthalat oder Polyvinylacetat erhalten werden. Die Tabletten können auch aus mehreren Schichten bestehen.

Entsprechend können Dragees durch Überziehen von analog den Tabletten hergestellten Kernen mit üblicherweise in Drageeüberzügen verwendeten Mitteln, beispielsweise Kollidon oder Schellack, Gummi arabicum, Talk, Titandioxid oder Zucker, hergestellt werden. Dabei kann auch die Dragee-

hülle aus mehreren Schichten bestehen, wobei die oben bei den Tabletten erwähnten Hilfsstoffe verwendet werden können.

Suspensionen mit den erfindungsgemäß zu verwendenden Wirkstoffen können zusätzlich geschmacksverbessernde Mittel, wie Saccharin, Cyclamat oder Zucker sowie z.B. Aromastoffe, wie Vanillin oder Orangenextrakt, enthalten. Sie können außerdem Suspendierhilfsstoffe, wie Natriumcarboxymethylcellulose, oder Schutzstoffe, wie p-Hydroxybenzoate, enthalten. Wirkstoffe enthaltende Kapseln können beispielsweise hergestellt werden, indem man den Wirkstoff mit einem inerten Träger, wie Milchzucker oder Sorbit, mischt und in Gelatinekapseln einkapselt.

Für die äußerliche Anwendung kommen insbesondere Pasten, Salben, Gele, Cremes, Lotionen, Puder, Lösungen oder Emulsionen und Sprays in Betracht.

Salben, Pasten, Cremes und Gele können neben dem oder den Wirkstoffen die üblichen Trägerstoffe enthalten, z.B. tierische und pflanzliche Fette, Wachse, Paraffine, Stärken, Tragant, Cellulosederivate, Polyäthylenglykole, Silicone, Bentonite, Kieselsäure, Talkum und Zinkoxid oder Gemische dieser Stoffe.

Puder und Sprays können neben dem Wirkstoff die üblichen Trägerstoffe enthalten, z.B. Milchzucker, Talkum, Kieselsäure, Aluminiumhydroxid, Calciumsilikat und Polyamidpulver oder Gemische dieser Stoffe. Sprays können zusätzlich die üblichen Treibmittel, z.B. Chlorfluorkohlenwasserstoffe, enthalten.

Lösungen und Emulsionen können neben dem oder den Wirkstoffen die üblichen Trägerstoffe, wie Lösungsmittel, Lösungsvermittler und Emulgatoren, z.B. Wasser, Äthylen-

alkohol, Isopropylalkohol, Äthylcarbonat, Äthylacetat, Benzylalkohol, Benzylbenzoat, Propylenglykol, 1,3-Butylenglykol, Dimethylformamid, Öle, insbesondere Baumwollsaatöl, Erdnußöl, Maiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Beispiele

1. cis- und trans-3-(4-tert.-Butyl-cyclohexyl-1)-2-methyl-1-(3'-methyl-1-piperidino)-propan

a) Synthese des aromatischen Amins:
In einem 2-l-Dreihalskolben werden 306 g 3-(p-tert.-Butyl-phenyl)-2-methyl-propanal vorgelegt und 72,5 g 98 %ige Ameisensäure zugetropft. Danach werden im Verlauf von drei Stunden 149 g 3-Methylpiperidin tropfenweise zugegeben. Die Temperatur erhöht sich dabei von 25 auf über 50°C. Anschließend wird das Reaktionsgemisch noch 12 Stunden am Rückfluß erhitzt, wobei $CO_2$-Entwicklung auftritt.

Nach beendeter Umsetzung wird unter vermindertem Druck fraktioniert destilliert. Nach einem Vorlauf von 47 g, der bis zu einer Temperatur von 130°C bei 0,2 Torr übergeht, destilliert das 3-(p-tert.-Butylphenyl)-2-methyl-1-(3'-methyl-1'-piperidino)-propan zwischen 130 bis 133°C bei einem Druck von 0,2 Torr über. Es werden 355 g erhalten, was einer Ausbeute, bezogen auf den eingesetzten Aldehyd, von 82,4 % entspricht.

b) Hydrierung zum Cyclohexanderivat:

In einem 3-l-Rollautoklav werden 309 g 3-(p-tert.--Butylphenyl)-2-methyl-1-(3'-methyl-1'-piperidino)-propan in 1000 g Dioxan mit Hilfe von 0,5 g $Ru_2O_3$-Hydrat hydriert. Bei einem Wasserstoffdruck von 100 bar und einer Temperatur von 120°C wird nach 3 Stunden kein Wasserstoff mehr aufgenommen. Anschließend wird bei 140°C und 120 bar $H_2$-Druck innerhalb von 12 Stunden ein Druckabfall von 35 bar festgestellt und nach Neueinstellen des Wasserstoffdruckes auf 160 bar wird bei einer Temperatur von 160°C im Verlauf von weiteren 10 Stunden nochmals ein Druckabfall von 10 registriert. Bei weiterem Anheben des Wasserstoffdruckes auf 160 bar und der Temperatur auf 180°C tritt keine Wasserstoffaufnahme mehr ein.

c) Fraktionierte Destillation:

Das Reaktionsgemisch, 1310 g, wird über eine Siebbodenkolonne mit 10 Siebböden bei einem Druck von 10 Torr fraktioniert destilliert. Nach dem Abdestillieren des Dioxans werden bei einem Rücklaufverhältnis von 1 : 5 folgende Fraktionen erhalten:

1. bis 185°C 27 g enthaltend 85 % cis- und 10 % trans-Verbindung (Rest von 5 % ist Vorlauf)
2. bei 185°C 114 g enthaltend 75 % cis- und 25 % trans-Verbindung
3. bis 186°C 60 g enthaltend 40 % cis- und 60 % trans-Verbindung
4. bis 187°C 55 g enthaltend 30 % cis- und 70 % trans-Verbindung

Der Destillationsrückstand von 40 g wird einer Kurzweg-Destillation bei 10 torr und 187°C unter-

worfen. Es werden 38 g der Zusammensetzung 92 % trans und nur 8 % cis erhalten. Es bleiben 2 g Destillationsrückstand.

Das cis-trans-Verhältnis wird jeweils durch Gaschromatographie bestimmt.

504 g nach der oben beschriebenen Methode hergestelltes 3-(4'-tert.-Butyl-cyclohexyl-1'-)-2-methyl-1-(3'-methyl-1'-piperidino)-propan, die 54 % cis- und 46 % trans-Cyclohexanderivat enthalten (insgesamt 425 g) werden an einer Kolonne mit 20 Siebböden fraktioniert. Es wird ein Rücklaufverhältnis von 1 : 3 eingestellt.

Bis zu einer Übergangstemperatur von 192°C gehen 70 g Vorlauf über. Danach werden die folgenden Fraktionen erhalten:

1. bis 197°C/$28_T$ 13 g enthaltend: 85 % cis und 3 % trans (Rest von 12 % ist Vorlauf)
2. bis 198°C/$26_T$ 52 g enthaltend: 93 % cis und 7 % trans
3. bis 197°C/$25_T$ 87 g enthaltend: 90 % cis und 10 % trans
4. bis 192°C/$22_T$ 82 g enthaltend: 85 % cis und 15 % trans
5. bis 196°C/$22_T$ 81 g enthaltend: 80 % cis und 20 % trans
6. bis 202°C/$28_T$ 87 g enthaltend: 30 % cis und 70 % trans
7. bis 196°C/$22_T$ 73 g enthaltend: 97 % trans und 3 % cis.

Der Destillationsrückstand von 27 g wird einer Kurzweg-Destillation unterworfen. Es werden 24 g

erhalten mit einem Gehalt an trans-Verbindung von über 98 %.

d) Herstellung des HBr-Salzes der reinen cis-Form: Die weitere Reinigung der Anfangs- und/oder Endfraktionen zu der reinen cis-Form wird durch Umkristallisation des Hydrobromids in Essigsäure/Wasser und im Falle der trans-Verbindung mit Hilfe von Chlorwasserstoff in Methanol vorgenommen:

25 g der oben erhaltenen ersten Fraktion werden mit 20 g wäßriger 48 %iger Bromwasserstoffsäure versetzt. Danach werden 50 g Essigsäure zugegeben und das gesamte Kristallisat durch Erwärmen in Lösung gebracht. Beim Abkühlen kristallisiert das Hydrobromid der cis-Form aus. Die NMR-Analyse bestätigt das Vorliegen der reinen cis-Form. Es werden 28 g mit einem Schmelzpunkt von 210°C erhalten.

C ber.: 64,15 % gef.: 64,5 %; H ber.: 10,77 %, gef.: 10,3 %

N ber.: 3,74 % gef.: 3,60 %; Br ber.: 20,34 %, gef.: 20,6 %

e) Herstellung des HCl-Salzes der reinen trans-Form: 35 g der oben erhaltenen Fraktion 5 werden in Methanol mit 60 g Chlorwasserstoff versetzt. Beim Abkühlen kristallisieren 28 g Hydrochlorid mit einem Schmelzpunkt von 168°C aus. Die NMR-Analyse bestätigt das Vorliegen der trans-Form.

2) cis- und trans-3-(4'-tert.-Butyl-cyclohexyl-1')-2--methyl-1-(3',5'-dimethyl-1'-piperidino)-propan

a) 361 g 3-(p-tert.-Butylphenyl)-2-methyl-propanal werden, wie unter Beispiel 1 beschrieben, mit 200 g 3,5-Dimethyl-piperidin und 86 g 98 %iger Ameisensäure umgesetzt. Die Reinigung des Amins wird durch fraktionierte Destillation bei einem Druck von 0,2 Torr über eine 10-Bodenkolonne bewirkt. Bei 136°C gehen 406 g reines Amin über.

b) Hydrierung zur Cyclohexanverbindung:
In einem Schüttelautoklaven mit einem Reaktionsvolumen von 250 ml werden 70 g des 3-(p-tert.--Butylphenyl)-2-methyl-1-(3',5'-dimethyl-1'-piperidino)-propans mit 80 g Dioxan versetzt. Als Hydrierkatalysator werden 0,5 g Rutheniumoxidhydrat zugegeben. Bei 100 bar Wasserstoff und 120°C werden innerhalb von 37 Stunden 358 bar nachgepresst.

Die fraktionierte Destillation des Reaktionsaustrages (139 g) liefert 64 g des Cyclohexanderivates, das zwischen 136 bis 146°C bei einem Druck von 3 Torr überdestilliert. Die gaschromatographische Analyse zeigt, daß es sich um ein Isomeren-Gemisch der beiden Formen cis und trans des Cyclohexanderivates handelt.

c) Fraktionierte Destillation:
562 g des aromatischen Amins, das in 1000 g Dioxan mit Hilfe von $Ru_2O_3$.Hydrat bei 140 bar Wasserstoffdruck und bei 120°C innerhalb von 20 Stunden hydriert wurde, wird bei der Fraktionierung über eine Kolonne mit 10 Siebböden bei

einem Rücklaufverhältnis von 1 : 5 getrennt, wie unter Beispiel 1 beschrieben.

Das über 90 %ige cis-Cyclohexanderivat geht bei einem Druck von 0,1 Torr zwischen 124-125°C über. Das auf über 90 % angereicherte trans-Cyclohexanderivat geht zwischen 129-131°C über.

Eine Wiederholung der fraktionierten Destillation über einer 80 cm langen mit Silberdrahtnetzwendeln bestückten Kolonne bei einem Druck von 12-13 Torr ermöglicht die Gewinnung von 154 g von auf über 90 % angereicherten cis-Verbindung. Ein Zwischenlauf von 136 g bestand aus einem Gemisch von 1 : 1 cis-trans-Cyclohexanderivat. An trans-angereichertem Cyclohexanderivat (Anreicherung auf über 90 %) werden 98 g gewonnen werden.

Der Zwischenlauf wird erneut zur Fraktionierung eingesetzt. Eine Rückstandsbildung trat nur in einem sehr geringen Umfang auf, bei der fraktionierten Destillation des cis-trans-Isomerengemisches bilden sich aus 390 g lediglich 2 g höhersiedende Anteile. Bei einem Druck von 12 Torr geht das cis-Derivat zwischen 193 bis 195°C und das angereicherte trans-Produkt zwischen 199-200°C über.

d) Kristallisation der reinen cis-Verbindung über das HCl-Salz:
20 g des auf über 90 % angereicherten cis-Produktes werden in 25 g methanolischer Chlorwasserstofflösung aufgenommen, wobei sich die Lösung bis fast zum Sieden erhitzt. Beim Abkühlen kristallisieren 12 g Hydrochlorid aus. Der Schmelz-

punkt des umkristallisierten cis-Cyclohexanderivats liegt bei 240°C. Eine Probe des Salzes, die mit KOH zerlegt wurde, zeigte bei der gaschromatographischen Analyse, daß es sich um trans-freie cis-Verbindung handelt.

e) Kristallisation der reinen trans-Verbindung über das HCl-Salz:
20 g der auf über 90 % angereicherten trans-Verbindung werden in 30 g äthanolischer Chlorwasserstofflösung aufgenommen. Beim Abkühlen kristallisiert das Hydrochlorid der reinen trans-Verbindung aus vom Fp = 202°C. Es werden 13 g erhalten. Die freie Base, die durch Zerlegen einer Salzprobe erhalten wird, zeigt bei der gaschromatographischen Untersuchung, daß es sich um cis-freie trans-Verbindung handelt. Die NMR-Analysen der beiden Kristallisate bestätigen die bereits durch die GC-Analyse erhaltenen Aussagen.

3) cis- und trans-3-(4'-tert.-Butyl-cyclohexyl-1')-2-methyl-1-(2',6'-cis-dimethyl-morpholyl)-propan

a) **Die Herstellung des 3-(p-tert.-Butylphenyl)-2-methyl-1-(2',6'-cis-dimethyl-morpholyl)-propans** erfolgt nach der unter Beispiel 1 beschriebenen Arbeitsweise durch Umsetzung von 3-(p-tert.-Butylphenyl)-2-methyl-propanal mit 2,6-cis-Dimethylmorpholin und Ameisensäure. Die Reinigung des aromatischen Amins erfolgt durch Destillation. Das Amin geht bei 3,0 Torr zwischen 170-175°C über.

b) Hydrierung zur Cyclohexanverbindung:
720 g des oben beschriebenen Morpholinderivates werden mit 500 g Dioxan versetzt. Als Hydrierkatalysator werden 0,5 g Rutheniumoxidhydrat zugegeben. Die Hydrierung wird bei einem Wasserstoffdruck von 120 bar und bei einer Temperatur von 140°C durchgeführt. Innerhalb von 24 Stunden werden bei der im 2,6-l-Rollautoklav durchgeführten Hydrierung insgesamt 130 bar Wasserstoff nachgepreßt.

c) Fraktionierte Destillation:
623 g des erhaltenen cis-trans-Isomerengemisches, das zu 60 % aus cis- und 40 % aus trans-Verbindung besteht, werden in einer 80 cm mit Silberdrahtnetzwendeln gefüllten Kolonne bei einem Druck von 3,0 Torr fraktioniert. Als Rücklaufverhältnis werden 1 : 5 eingestellt. Die cis-Verbindung geht zwischen 144 bis 145°C bei 3,0 Torr über. Die Anreicherung der cis-Verbindung in den ersten drei Fraktionen zu je etwa 70 g erfolgt auf über 90 %. Die letzten drei Fraktionen enthalten die angereicherte trans-Verbindung. 71 g, 54 g und 38 g. Der trans-Anteil liegt über 85 %.

d) Kristallisation des reinen cis- und trans-HCl-Salzes:

Nach der unter Beispiel 1 und 2 beschriebenen Methode läßt sich das reine Hydrochlorid der cis-Verbindung mit äthanolischer Chlorwasserstofflösung gewinnen. Fp. 161°C.

Die reine trans-Verbindung bildet ein Hydrochlorid mit einem Schmelzpunkt von 199°C.

Die gaschromatographische Analyse, der aus beiden Salzen durch Umsetzung mit KOH hergestellten freien Basen bestätigt die Reinheit der Basen. Das so vorgereinigte cis-Cyclohexanderivat enthält weniger als 0,5 % trans-Produkt; das trans-Produkt enthält weniger als 1 % cis-Verbindung. Die NMR-Analyse bestätigt den Befund der GC-Analyse.

Beispiele für pharmazeutische Zubereitungen:

1. Beispiel für Tabletten

| | |
|---|---|
| 1. Wirkstoff | 200 mg |
| 2. Polyvinylpyrrolidon (mittl. M.G. 25 000) | 20 mg |
| 3. Polyäthylenglykol (mittl. M.G. 4 000) | 14 mg |
| 4. Hydroxypropylmethylcellulose | 40 mg |
| 5. Talkum | 4 mg |
| 6. Magnesiumstearat | 2 mg |
| | 280 mg |

Der Wirkstoff wird mit Polyvinylpyrrolidon in 10 %iger wäßriger Lösung befeuchtet, durch ein Sieb mit der lichten Maschenweite 1,0 mm getrieben und bei 50°C getrocknet. Dieses Granulat wird mit Polyäthylenglykol (mittl. M.G. 4 000), Hydroxypropylmethylcellulose, Talkum und Magnesiumstearat vermischt und zu Tabletten à 280 mg verpreßt.

2. Beispiel für Dragees

| | |
|---|---|
| 1. Wirkstoff | 150 mg |
| 2. Lactose | 60 mg |
| 3. Maisstärke | 30 mg |
| 4. Polyvinylpyrrolidon | 4 mg |
| 5. Magnesiumstearat | 1 mg |
| | 245 mg |

Die Mischung der Wirkstoffsubstanz mit Lactose und Maisstärke wird mit einer 8 %igen wäßrigen Lösung des Polyvinylpyrrolidons durch Sieb 1,5 mm granuliert, bei 50°C getrocknet und nochmals durch Sieb 1,0 mm gerieben. Das so erhaltene Granulat wird mit Magnesiumstearat gemischt und zu Drageekernen verpreßt. Die erhaltenen Drageekerne werden in üblicher Weise mit einer Hülle überzogen, die im wesentlichen aus Zucker und Talkum besteht.

3. Beispiel für Creme mit 2 % Wirkstoff

| | |
|---|---|
| 1. Wirkstoff | 2,0 g |
| 2. Glycerinmonostearat | 10,0 g |
| 3. Cetylalkohol | 5,0 g |
| 4. Polyäthylenglykol-400-stearat | 10,0 g |
| 5. Polyäthylenglykol-sobitan-monostearat | 10,0 g |
| 6. Propylenglykol | 6,0 g |
| 7. p-Hydroxybenzoesäuremethylester | 0,2 g |
| 8. Demineralisiertes Wasser | ad 100,0 g |

Der feinst gepulverte Wirkstoff wird im Propylenglykol suspendiert und die Suspension in die auf 65°C erwärmte Schmelze aus Glycerinmonostearat, Cetylalkohol, Polyäthylenglykol-400-stearat und Polyäthylenglykol-sorbitanmonostearat gerührt. In diese Mischung

wird die 70°C heiße Lösung des p-Hydroxybenzoesäuremethylesters in Wasser emulgiert. Nach dem Erkalten wird die Creme über eine Kolloidmühle homogenisiert und in Tuben abgefüllt.

4. Beispiel für Puder mit 2 % Wirkstoff

| | |
|---|---|
| 1. Wirkstoff | 2,0 g |
| 2. Zinkoxid | 10,0 g |
| 3. Magnesiumoxid | 10,0 g |
| 4. Hochdisperses Silisiumoxid | 2,5 g |
| 5. Magnesiumstearat | 1,0 g |
| 6. Talkum | 75,5 g |

Der Wirkstoff wird auf einer Luftstrahlmühle mikronisiert und mit den anderen Bestandteilen homogen vermischt.

Die Mischung wird durch Sieb Nr. 7 geschlagen und in Polyäthylenbehälter mit Streueinsatz abgefüllt.

D

Patentansprüche

1. Reines trans-3-(4'-tert.-Butyl-cyclohexyl-1')-2-methyl-1-(3'-methyl-1'-piperidino)-propan.

2. Reines trans-3-(4'-tert.-Butyl-cyclohexyl-1')-2-methyl-1-(3',5'-dimethyl-1'-piperidino)-propan.

3. Reines trans-3-(4'-tert.-Butyl-cyclohexyl-1')-2-methyl-1-(2',6'-cis-dimethylmorpholino)-propan.

4. Verfahren zur Herstellung der reinen trans-3-(4'-tert.-Butyl-cyclohexyl-1')-2-methyl-1-(3'-methylpiperidino, 3',5'-dimethylpiperidino und 2',6'-cis-dimethylmorpholino)-propane aus den bei der Hydrierung der entsprechenden 1,4-disubstituierten Phenylverbindung erhaltenen cis-trans-Gemischen, dadurch gekennzeichnet, daß man nach dem Abdestillieren des für die Hydrierung verwendeten Lösungsmittels das Isomerengemisch durch eine fraktionierte Destillation bei einem Druck am Kolonnenkopf von 0,05 bis 50 torr an einer 10- bis 100-bödigen Kolonne unter einem Rücklaufverhältnis von 1 : 1 bis 1 : 100 bei den bei der Destillation sich einstellenden Temperaturen auf einen trans-Anteil von über 85 % anreichert und das reine trans-Isomere nach Umwandlung in ein Säureadditionssalz einer Halogenwasserstoffsäure und Kristallisation in einem organischen Lösungsmittel auskristallisieren läßt.

5. Antimykotisches Mittel, gekennzeichnet durch einen Gehalt an einer reinen trans-Verbindung gemäß Patentansprüchen 1 bis 3 oder deren physiologisch verträgliches Säureadditionssalz als Wirkstoff neben üblichen Träger- und Verdünnungsmitteln.

6. Verwendung der reinen trans-Verbindungen gemäß Patentansprüchen 1 bis 3 oder deren physiologisch verträgliche Säureadditionssalzen bei der Bekämpfung von Dermatomykosen.

0019764

Europäisches Patentamt

**EUROPÄISCHER RECHERCHENBERICHT**

Nummer der Anmeldung

EP 80 10 2509

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| D | DE - A - 2 752 135 (HOFFMANN- LA ROCHE)<br>* Ansprüche *<br>-- | 1-6 |
| A | DE - B - 1 214 471 (BASF)<br>* Spalten 1,2 *<br>-- | 1-3 |
| A | FR - A - 2 374 316 (BASF)<br>* Seiten 15-17 *<br>-- | 1-3 |
| A | GB - A - 988 630 (BASF)<br>* Spalten 1,2 *<br>-- | 1-3 |
| A | NL - A - 202 320 (N.V. PHILIPS)<br>* Spalten 2,3 *<br>-- | 1-3 |
| P | EP - A - 0 008 686 (HOFFMANN- LA ROCHE)<br>* Ansprüche *<br>-- | 1-3 |
| P | DE - A - 2 830 127 (BASF)<br>* Ansprüche *<br>---- | 1-3 |

**KLASSIFIKATION DER ANMELDUNG (Int.Cl. ³)**

C 07 D 211/14
265/30
A 61 K 31/445
31/535

**RECHERCHIERTE SACHGEBIETE (Int. Cl. ³)**

C 07 D 211/14
265/30
A 61 K 31/445
31/535

**KATEGORIE DER GENANNTEN DOKUMENTE**

X: von besonderer Bedeutung
A: technologischer Hintergrund
O: nichtschriftliche Offenbarung
P: Zwischenliteratur
T: der Erfindung zugrunde liegende Theorien oder Grundsätze
E: kollidierende Anmeldung
D: in der Anmeldung angeführtes Dokument
L: aus andern Gründen angeführtes Dokument
&: Mitglied der gleichen Patentfamilie, übereinstimmendes Dokument

X Der vorliegende Recherchenbericht wurde für alle Patentansprüche erstellt.

| Recherchenort | Abschlußdatum der Recherche | Prüfer |
|---|---|---|
| Den Haag | 12-08-1980 | BRIGHENTI |